(19) Europäisches Patentamt / European Patent Office / Office européen des brevets

(11) **EP 3 480 189 B1**

(12) **EUROPEAN PATENT SPECIFICATION**

(45) Date of publication and mention of the grant of the patent:
**24.11.2021 Bulletin 2021/47**

(51) Int Cl.:
***C07D 301/19*** *(2006.01)*     ***C07D 303/04*** *(2006.01)*

(21) Application number: **17820034.1**

(86) International application number:
**PCT/JP2017/023133**

(22) Date of filing: **23.06.2017**

(87) International publication number:
**WO 2018/003677 (04.01.2018 Gazette 2018/01)**

(54) **METHOD FOR PRODUCING PROPYLENE OXIDE**

VERFAHREN ZUR HERSTELLUNG VON PROPYLENOXID

PROCÉDÉ DE FABRICATION D'OXYDE DE PROPYLÈNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2016 JP 2016130588**
           **27.12.2016 JP 2016253298**

(43) Date of publication of application:
**08.05.2019 Bulletin 2019/19**

(73) Proprietor: **SUMITOMO CHEMICAL COMPANY, LIMITED**
**Chuo-ku**
**Tokyo 104-8260 (JP)**

(72) Inventors:
- **ISHIHARA, Shinjiro**
  **Ichihara-shi**
  **Chiba 299-0195 (JP)**
- **HASHIMOTO, Yasuhiro**
  **Aichi 4740061 (JP)**
- **SUZUKI, Tetsuo**
  **Ichihara-shi**
  **Chiba 299-0195 (JP)**

(74) Representative: **Winter, Brandl - Partnerschaft mbB**
**Alois-Steinecker-Straße 22**
**85354 Freising (DE)**

(56) References cited:
**WO-A1-2014/003732**     **JP-A- 2005 089 378**
**JP-A- 2005 089 411**     **JP-A- 2005 120 076**
**JP-A- 2011 201 821**

**Description**

**Technical Field**

The present invention relates to a method for producing propylene oxide.

**Background Art**

In patent literature 1, a method for producing propylene oxide comprising an oxidation step of oxidizing cumene to obtain cumene hydroperoxide, an epoxidation step of causing a reaction between the cumene hydroperoxide obtained in the oxidation step and propylene to obtain propylene oxide and cumyl alcohol, and a hydrogenation step of hydrogenating the cumyl alcohol obtained in the epoxidation step to obtain cumene and recycling the cumene to the oxidation step as a raw material in the oxidation step, is described. In the present specification, cumyl alcohol denotes 2-phenyl-2-propanol.

**Citation List**

**Patent Literature**

Patent Literature 1: Japanese Unexamined Patent Publication No. 2005-97212 (laid-open on April 14, 2005).

**Summary of Invention**

**Technical Problem**

In the above manufacturing method, it is required to improve total efficiency of the manufacturing step including energy efficiency and reaction efficiency.
In order to solve the above problem in the method for producing propylene oxide through recycling of cumene for use as a raw material, the present invention provides a method for further efficiently producing propylene oxide through improvement in the oxidation reaction rate of cumene in the oxidation step and the conversion ratio of cumene hydroperoxide in the epoxidation step.

**Solution to Problem**

In order to solve the problem, the present invention provides the following methods.

<1> A method for producing propylene oxide comprising the following steps 1) to 5):

1) Oxidation step: a step of obtaining a reaction mixture containing cumene hydroperoxide by contacting a mixture containing cumene and cymene with an oxygen-containing gas to cause a reaction between cumene in the mixture and oxygen in the gas;
2) Epoxidation step: a step of obtaining a reaction mixture containing propylene oxide and cumyl alcohol by contacting the reaction mixture containing cumene hydroperoxide obtained in the oxidation step with propylene in the presence of a catalyst to cause a reaction between cumene hydroperoxide in the reaction mixture and propylene;
3) Separation step: a step of obtaining a residual mixture containing cumyl alcohol by separating a mixture containing propylene oxide from the reaction mixture containing propylene oxide and cumyl alcohol obtained in the epoxidation step;
4) Cumene conversion step: a step of obtaining a reaction mixture containing cumene by converting cumyl alcohol in the residual mixture containing cumyl alcohol obtained in the separation step into cumene; and
5) Cumene recovery step: a step of obtaining a recovery mixture containing cumene and cymene with a cymene concentration in a steady state of 100 ppm by weight or more and 5000 ppm or less by weight (the cymene concentration is calculated based on the weight of the recovery mixture containing cumene and cymene obtained in the cumene recovery step) by distilling the reaction mixture containing cumene obtained in the cumene conversion step, wherein the distillation is performed under a condition for removing 0.025 wt% or more of cymene contained in the reaction mixture containing cumene obtained in the cumene conversion step (the weight ratio of cymene is calculated based on the weight of cymene contained in the reaction mixture containing cumene obtained in the cumene conversion step); wherein, at least a part of the mixture containing cumene and cymene in the oxidation step is the recovery mixture containing cumene and cymene obtained in the cumene recovery step.

At least a part of the mixture containing cumene and cymene in the oxidation step is the recovery mixture containing cumene and cymene obtained in the cumene recovery step.

Preferred embodiments are found in the dependent claims.

**Advantageous Effects of Invention**

According to the present invention, in a method for producing propylene oxide by recycling cumene for use as a raw material, the oxidation reaction rate of cumene in the oxidation step and the conversion ratio of cumene hydroperoxide in the epoxidation step can be improved to produce propylene oxide more efficiently.

**Description of Embodiments**

[Method for producing propylene oxide]

The present invention provides a method for producing propylene oxide comprising the following steps 1) to 5):

1) Oxidation step: a step of obtaining a reaction mixture containing cumene hydroperoxide by contacting a mixture containing cumene and cymene with an oxygen-containing gas to cause a reaction between cumene in the mixture and oxygen in the gas;

2) Epoxidation step: a step of obtaining a reaction mixture containing propylene oxide and cumyl alcohol by contacting the reaction mixture containing cumene hydroperoxide obtained in the oxidation step with propylene in the presence of a catalyst to cause a reaction between cumene hydroperoxide in the reaction mixture and propylene;

3) Separation step: a step of obtaining a residual mixture containing cumyl alcohol by separating a mixture containing propylene oxide from the reaction mixture containing propylene oxide and cumyl alcohol obtained in the epoxidation step;

4) Cumene conversion step: a step of obtaining a reaction mixture containing cumene by converting cumyl alcohol in the residual mixture containing cumyl alcohol obtained in the separation step into cumene; and

5) Cumene recovery step: a step of obtaining a recovery mixture containing cumene and cymene with a cymene concentration in a steady state of 100 ppm by weight and 5000 ppm or less by weight (the cymene concentration is calculated based on the weight of the recovery mixture containing cumene and cymene obtained in the cumene recovery step) by distilling the reaction mixture containing cumene obtained in the cumene conversion step, wherein the distillation is performed under a condition for removing 0.025 wt% or more of cymene contained in the reaction mixture containing cumene obtained in the cumene conversion step (the weight ratio of cymene is calculated based on the weight of cymene contained in the reaction mixture containing cumene obtained in the cumene conversion step); wherein, at least a part of the mixture containing cumene and cymene in the oxidation step is the recovery mixture containing cumene and cymene obtained in the cumene recovery step.

At least a part of the mixture containing cumene and cymene in the oxidation step is the recovery mixture containing cumene and cymene obtained in the cumene recovery step.

In an aspect, the cumene conversion step is a step of obtaining a reaction mixture containing cumene by dehydrating cumyl alcohol in the residual mixture containing cumyl alcohol obtained in the separation step in the presence of a catalyst to obtain a mixture containing $\alpha$-methylstyrene, and then contacting the mixture containing $\alpha$-methylstyrene with hydrogen in the presence of a catalyst to cause a reaction between $\alpha$-methylstyrene in the mixture and hydrogen.

In another aspect, the cumene conversion step is a step of obtaining a reaction mixture containing cumene by contacting the residual mixture containing cumyl alcohol obtained in the separation step with hydrogen in the presence of a catalyst to cause a reaction between cumyl alcohol in the residual mixture and hydrogen.

In the method for producing propylene oxide by recycling of cumene for use as a raw material, cymene is produced as a by-product. It was found that cymene is a component to be accumulated in a system, whose concentration increases over time in continued recycling, and that propylene oxide can be efficiently produced by controlling the cymene concentration at a specified level. Cymene is the general term for o-cymene, m-cymene and p-cymene, and the cymene concentration is the total concentration of o-cymene, m-cymene and p-cymene. The cymene concentration can be measured by gas chromatography.

Each of the steps is described in detail as follows.

1) Oxidation step

The oxidation step of the present invention is a step of obtaining a reaction mixture containing cumene hydroperoxide by contacting a mixture containing cumene and cymene with an oxygen-containing gas to cause a reaction between cumene in the mixture and oxygen in the gas.

The cymene concentration of the mixture containing cumene and cymene to be used in the oxidation step is preferably 100 ppm by weight or more, more preferably 110 ppm by weight or more, still more preferably 120 ppm by weight or more, particularly preferably 130 ppm by weight or more such that the energy required for distillation does not increase excessively in the cumene recovery step. The cymene concentration of the mixture containing cumene and cymene to be supplied to the oxidation step is preferably less than 10000 ppm by weight, more preferably 5000 ppm by weight or less, still more preferably 3000 ppm by weight or less, particularly preferably 1000 ppm by weight or less in order to more efficiently produce cumene hydroperoxide in the oxidation step and more efficiently produce propylene oxide in the epoxidation step. The cymene concentration is calculated based on the weight of the mixture containing cumene and cymene for use in the oxidation step.

As described below, in the present invention, at least a part of the mixture containing cumene and cymene in the oxidation step is the recovery mixture containing cumene and cymene with a cymene concentration of 100 ppm by weight or more and 5000 ppm or less by weight obtained in the following 5) cumene recovery step.

As described below, in the present invention, per 100 wt% of a mixture containing cumene and cymene for use in an oxidation step, it is more preferable that the concentration of a recovery mixture containing cumene and cymene with a concentration of cymene obtained in the cumene recovery step of 100 ppm by weight or more and less than 10000 ppm by weight is 50 wt% or more, still more preferable that the concentration of a recovery mixture containing cumene and cymene with a concentration of cymene obtained in the cumene recovery step of 100 ppm by weight or more and less than 10000 ppm by weight is 75 wt% or more, and further more preferable that the concentration of a recovery mixture containing cumene and cymene with a concentration of cymene obtained in the cumene recovery step of 100 ppm by weight or more and less than 10000 ppm by weight is 90 wt% or more.

Examples of the oxygen-containing gas for use in the oxidation step include oxygen, air, and air with condensed oxygen.

The cumene hydroperoxide content of the reaction mixture containing cumene hydroperoxide is preferably 5 to 80 wt%, more preferably 5 to 60 wt%, still more preferably 5 to 40 wt%, per 100 wt% of the reaction mixture.

In the oxidation step, cumene is oxidized by oxygen to produce cumene hydroperoxide. The oxidation may be performed in the presence of an alkaline aqueous solution. The alkaline aqueous solution may be any aqueous solution with alkaline nature, and a fresh alkaline solution, an aqueous solution recovered from the oxidation step, an aqueous solution prepared by mixing an aqueous solution recovered from the oxidation step with a fresh alkaline solution, or the like is used. Examples of the fresh alkaline aqueous solution for use include an aqueous solution prepared by dissolving in water alkaline metal compounds such as NaOH and KOH, alkaline earth metal compounds such as $Mg(OH)_2$ and $Ca(OH)_2$, alkaline metal carbonates such as $Na_2CO_3$ and $NaHCO_3$, or ammonia and $(NH_4)_2CO_3$, or alkaline metal ammonium carbonates. The recovery of the aqueous solution in the oxidation step can be performed by separating a liquid phase produced in the oxidation step into an oil phase and an aqueous phase, and recovering the aqueous phase. The oxidation reaction temperature is usually 50 to 200°C, preferably 60 to 180°C, more preferably 70 to 150°C. The reaction pressure is usually between atmospheric pressure and 5000 kPaG, preferably 10 to 2000 kPaG, more preferably 20 to 1000 kPaG.

Cumene, cumyl alcohol, acetophenone, ethylbenzene, cymene, etc., are contained in the reaction mixture containing cumene hydroperoxide, as components thereof other than cumene hydroperoxide.

2) Epoxidation step

The epoxidation step of the present invention is a step of obtaining a reaction mixture containing propylene oxide and cumyl alcohol by contacting the reaction mixture containing cumene hydroperoxide obtained in the oxidation step with propylene in the presence of a catalyst to cause a reaction between cumene hydroperoxide in the reaction mixture and propylene. In the present specification, the reaction for producing propylene oxide through a reaction between cumene hydroperoxide and propylene is referred to as "epoxidation reaction" in some cases.

From the viewpoint of producing propylene oxide at a high yield, it is preferable that the epoxidation step is performed with use of a solid catalyst as the catalyst (hereinafter referred to as "epoxidation catalyst" in some cases), and it is more preferable that the step is performed in the presence of a catalyst comprising a titanium-containing silicon oxide. The catalyst comprising a titanium-containing silicon oxide contains titanium chemically bonded to a silicon oxide, and examples thereof include a titanium compound supported on a silica support, a titanium compound combined with a silicon oxide by a co-precipitation process or a sol-gel process, and a titanium-containing zeolite compound. Preferred examples of the catalyst comprising a titanium-containing silicon oxide include catalysts described in Japanese Patent No. 3731384, Japanese Patent No. 3797107 and the like, catalysts described in US 2005014960, US 2007260074 and the like, Ti-MCM-41 described in U.S. Patent No. 5783167 and the like, Ti-MCM-48 described in Japanese Unexamined Patent Publication No. H7-300312 and the like, Ti-HMS described in Nature 368 (1994) p. 321, CN101348472B, CN101307039B, CN101279960B, CN102311363B, CN102872847B, CN103030611B and the like, Ti-SBA-15, TS-1 and TS-2 described in Chemistry of Material 14, 2002, p. 1657 and the like, Ti-MWW described in Chemistry Letters 2000, p. 774 and the like, and a precursor thereof (e.g., Japanese Unexamined Patent Publication No. 2003-32745).

In the present invention, the reaction mixture containing cumene hydroperoxide obtained in the oxidation step to be used as a raw material in the epoxidation step may be a purified or unpurified product which is diluted or condensed.

The epoxidation reaction is performed by contacting propylene and cumene hydroperoxide with a catalyst. The epoxidation reaction may be performed in a liquid phase, using a solvent. The solvent for use in the epoxidation reaction is required to be a liquid under the temperature and pressure conditions during the epoxidation reaction, and to be substantially inert to the raw materials and the products of the reaction. The solvent for use in the epoxidation reaction may be a material present in the reaction mixture containing cumene hydroperoxide obtained in the oxidation step, and for example, in the case where the reaction mixture obtained in the oxidation step contains cumene, the cumene can be the solvent for use in the epoxidation reaction. Examples of the useful solvents other than cumene include monocyclic aromatic compounds such as benzene, toluene, chlorobenzene and orthodichlorobenzene, and alkanes such as octane, decane and dodecane.

The epoxidation reaction temperature is usually 0 to 200°C, preferably 25 to 200°C, more preferably 50 to 150°C. The epoxidation reaction pressure is controlled such that the reaction mixture can be maintained in a liquid state, and is usually 100 to 10000 kPaG, preferably 500 to 8000 kPaG.

The epoxidation reaction can be favorably performed in a slurry or a fixed bed mode. In a large-scale industrial operation, it is preferable to use a fixed bed. Further, the epoxidation reaction may be performed by a batch process, a semi-continuous process, a continuous process, etc.

The propylene oxide content of the reaction mixture containing propylene oxide and cumyl alcohol obtained in the epoxidation step is preferably 1 to 31 wt%, more preferably 1 to 23 wt%, particularly preferably 1 to 16 wt%, per 100 wt% of the reaction mixture. The cumyl alcohol content is preferably 5 to 80 wt%, more preferably 5 to 60 wt%, particularly preferably 5 to 40 wt%, per 100 wt% of the reaction mixture.

In the epoxidation step, the molar ratio of propylene to be supplied to the epoxidation reaction to cumene hydroperoxide is usually 2/1 to 50/1, preferably 3/1 to 30/1, more preferably 4/1 to 15/1.

In the case where the reaction mixture containing cumene hydroperoxide to be used in the epoxidation step contains cymene, the cymene concentration of the reaction mixture is preferably 100 ppm by weight or more, more preferably 110 ppm by weight or more, still more preferably 120 ppm by weight or more, particularly preferably 130 ppm by weight or more such that the energy required for distillation does not increase excessively in the cumene recovery step. The cymene concentration of the reaction mixture containing cumene hydroperoxide to be supplied to the epoxidation step is preferably less than 10000 ppm by weight, more preferably 5000 ppm by weight or less, still more preferably 3000 ppm by weight or less, particularly preferably 1000 ppm by weight or less in order to more efficiently produce propylene oxide in the epoxidation step. The cymene concentration is calculated based on the weight of the reaction mixture containing cumene hydroperoxide to be used in the epoxidation step.

Alternatively, unreacted propylene may be recovered after the epoxidation step so as to further perform a step of recycling the propylene as the raw material in the epoxidation step.

3) Separation step

The separation step of the present invention is a step of separating a mixture from the reaction mixture containing propylene oxide and cumyl alcohol obtained in the epoxidation step, the separated mixture containing the propylene oxide, so that a residual mixture containing the cumyl alcohol is obtained.

Examples of the method for separating the reaction mixture containing propylene oxide and cumyl alcohol obtained in the epoxidation step into a mixture containing propylene oxide and a residual mixture containing cumyl alcohol include distillation. As the conditions for distillation, the pressure is usually 100 to 5000 kPaG, preferably 100 to 3000 kPaG, and the column top temperature is usually —50 to 150°C, preferably —40 to 130°C. Examples of the distillation process include a method using a distillation column, and a distillation method using a plurality of distillation columns may be employed.

The content of propylene oxide in a mixture containing the separated propylene oxide is usually 99 wt% or more per 100 wt% of the mixture.

On the other hand, the concentration of cumyl alcohol in a residual mixture containing cumyl alcohol is preferably 5 to 80 wt%, more preferably 5 to 60 wt%, particularly preferably 5 to 40 wt%, per 100 wt% of the residual mixture.

Cumene, acetophenone, ethylbenzene, phenol, cymene, etc., are contained in the residual mixture containing cumyl alcohol as components other than cumyl alcohol contained therein.

Propylene oxide purification step

In an embodiment, the method for producing propylene oxide of the present invention includes a propylene oxide purification step. The propylene oxide purification step of the present invention is a step of obtaining purified propylene oxide by distilling the mixture containing propylene oxide separated in the separation step.

In the mixture containing propylene oxide obtained in the separation step, water, hydrocarbons and oxygen-containing compounds are usually contained as impurities. Examples of the hydrocarbons include hydrocarbons having 3 to 7 carbons. Examples of the oxygen-containing compounds include methanol, acetaldehyde, acetone, propionaldehyde, and methyl formate.

As the method for removing the impurities, known distillation techniques may be appropriately combined, and it is preferred that purification is performed through combination of extractive distillation with an extracting agent of hydrocarbons having 7 to 10 carbon atoms and other distillation, from the viewpoint of efficiently removing water, hydrocarbons, and oxygen-containing compounds.

Examples of the extracting agents of hydrocarbons having 7 to 10 carbon atoms include straight-chain saturated hydrocarbons such as n-heptane, n-octane, n-nonane, and n-decane, and branched saturated hydrocarbons such as 2,2-dimethylpentane, 2,3-dimethylpentane, 2,2-dimethylhexane and 2,3-dimethylhexane. These extracting agents may be used either singly or as a mixture of those compounds.

The types and the operation conditions of the extractive distillation column and the other distillation column, and the amount of the extracting agent used are appropriately determined depending on the product quality required.

4) Cumene conversion step

The cumene conversion step of the present invention is a step of obtaining a reaction mixture containing cumene, by converting cumyl alcohol in the residual mixture containing cumyl alcohol obtained in the separation step into cumene.

In an aspect, the cumene conversion step is a step of obtaining a reaction mixture containing cumene, by dehydrating cumyl alcohol in the residual mixture containing cumyl alcohol obtained in the separation step in the presence of a catalyst so as to obtain a mixture containing $\alpha$-methylstyrene, and then subsequently contacting the mixture containing $\alpha$-methylstyrene with hydrogen in the presence of a catalyst so as to cause a reaction between $\alpha$-methylstyrene in the mixture and hydrogen. In the present aspect, the step of obtaining a mixture containing $\alpha$-methylstyrene by dehydrating cumyl alcohol in the residual mixture obtained in the separation step in the presence of a catalyst is referred to as "dehydration step", and the step of obtaining a reaction mixture containing cumene by contacting the mixture containing $\alpha$-methylstyrene with hydrogen in the presence of a catalyst so as to cause a reaction between $\alpha$-methylstyrene in the mixture and hydrogen is referred to as "hydrogenation step", in some cases.

In another aspect, the cumene conversion step of the present invention is a step of obtaining a reaction mixture containing cumene by contacting the residual mixture containing cumyl alcohol obtained in the separation step with hydrogen in the presence of a catalyst so as to cause a reaction between cumyl alcohol in the residual mixture and hydrogen. The cumene conversion step is referred to as "hydrogenolysis step" in some cases.

The case where a cumene conversion step comprises the dehydration step and the hydrogenation step is described as follows.

The dehydration step is a step of obtaining a mixture containing $\alpha$-methylstyrene by dehydrating cumyl alcohol in the residual mixture containing cumyl alcohol obtained in the separation step in the presence of a catalyst.

Examples of the catalyst for use in the dehydration step (hereinafter referred to as "dehydration catalyst" in some cases) include acids such as sulfuric acid, phosphoric acid and p-toluene sulfonic acid, and metal oxides such as activated alumina, titania, zirconia, silica-alumina and zeolite. From the viewpoint of improving the reaction efficiency, it is preferable that the step is performed in the presence of a solid catalyst, and it is more preferable that activated alumina is used.

The dehydration reaction in the dehydration step is usually performed by contacting cumyl alcohol with a dehydration catalyst. In an embodiment, since the dehydration reaction is followed by the hydrogenation reaction in the hydrogenation step, cumyl alcohol may be contacted with a dehydration catalyst in the presence of hydrogen. The dehydration reaction can be performed in a liquid phase by using a solvent. The solvent is required to be substantially inert to the reaction raw materials and the products. The solvent may be a substance present in the residual mixture containing cumyl alcohol to be used. For example, in the case where the residual mixture containing cumyl alcohol contains cumene, the cumene can be used as the solvent without use of other solvent. The dehydration reaction temperature is usually 50 to 450°C, preferably 150 to 300°C. The dehydration pressure is usually 10 to 10000 kPaG, preferably 500 to 4000 kPaG, more preferably 1000 to 2000 kPaG.

The hydrogenation step is a step of obtaining a reaction mixture containing cumene by contacting the mixture containing $\alpha$-methylstyrene obtained in the dehydration step with hydrogen in the presence of a catalyst so as to cause

a reaction between $\alpha$-methylstyrene in the mixture and hydrogen.

Examples of the catalyst used in the hydrogenation step (hereinafter, referred to as "hydrogenation catalyst" in some cases) include catalysts comprising metals in group 10 or group 11 in the periodic table, specifically including catalysts comprising nickel, catalysts comprising palladium, catalysts comprising platinum, and catalyst comprising copper. From the viewpoints of inhibition of the nucleus hydrogenation reaction of an aromatic ring and the high yield, catalysts comprising nickel, catalysts comprising palladium or catalysts comprising copper are preferred. As the catalysts comprising nickel, nickel, nickel-alumina, nickel-silica and nickel-carbon are preferred; as the catalysts comprising palladium, palladium-alumina, palladium-silica and palladium-carbon are preferred; and as the catalysts comprising copper, copper, Raney copper, copper-chromium, copper-zinc, copper-chromium-zinc, copper-silica and copper-alumina are preferred. These catalysts may be used singly or a plurality thereof may be used.

The hydrogenation reaction in the hydrogenation step is performed by contacting $\alpha$-methylstyrene and hydrogen with a hydrogenation catalyst. In an embodiment, the dehydration reaction is followed by the hydrogenation reaction, and in this aspect, a part of water generated in the dehydration reaction may be separated by an oil-water separator or may not be separated to be contacted with a hydrogenation catalyst together with $\alpha$-methylstyrene. Although the amount of hydrogen required for the hydrogenation reaction may be an equimolar amount of $\alpha$-methylstyrene, an excessive amount of hydrogen is required because components other than $\alpha$-methylstyrene to consume hydrogen are usually contained in the raw material. Since the reaction proceeds faster as the partial pressure of hydrogen is increased, the molar ratio of hydrogen/$\alpha$-methylstyrene is adjusted to usually 1/1 to 20/1, preferably 1/1 to 10/1, more preferably 1/1 to 3/1. The excessive amount of hydrogen remaining after the hydrogenation reaction may be recycled for use after separation from the reaction liquid. The hydrogenation reaction may be performed in a liquid phase using a solvent, or in a gas phase. The solvent is required to be substantially inert to the reaction raw materials and the products. The solvent may be a substance present in the mixture containing $\alpha$-methylstyrene. For example, in the case where the mixture containing $\alpha$-methylstyrene contains cumene, the cumene can be used as the solvent without use of other solvent. The hydrogenation reaction temperature is usually 0 to 500°C, preferably 30 to 400°C, more preferably 50 to 300°C. The hydrogenation reaction pressure is usually 100 to 10000 kPaG, preferably 500 to 4000 kPaG, more preferably 1000 to 2000 kPaG.

The dehydration reaction and the hydrogenation reaction can be favorably performed in a slurry or a fixed bed mode. In a large-scale industrial operation, it is preferable to use a fixed bed. Further, the dehydration reaction and the hydrogenation reaction may be performed by a reaction mode such as a batch process, a semi-continuous process, and a continuous process. The dehydration reaction and the hydrogenation reaction may be performed either in separate reactors or in a single reactor. Among continuous reactors including adiabatic reactors and isothermal reactors, adiabatic reactors are preferred because isothermal reactors require equipment for heat removal.

The hydrogenolysis step is a step of obtaining a reaction mixture containing cumene by contacting the residual mixture containing cumyl alcohol obtained in the separation step with hydrogen in the presence of a catalyst to cause a reaction between cumyl alcohol in the residual mixture and hydrogen. The hydrogenolysis reaction is performed by contacting the residual mixture containing cumyl alcohol and hydrogen with a catalyst. Examples of the catalyst for use in the hydrogenolysis step (hereinafter, referred to as "hydrogenolysis catalyst" in some cases) include catalysts comprising metals in group 10 or group 11, or group 12 in the periodic table, specifically including catalysts comprising cobalt, catalysts comprising nickel, catalysts comprising palladium, catalysts comprising copper, and catalysts comprising zinc. From the viewpoint of inhibiting formation of by-products, catalysts comprising nickel, catalysts comprising palladium or catalysts comprising copper are preferred. Examples of the catalysts comprising nickel include nickel, nickel-alumina, nickel-silica and nickel-carbon; examples of the catalysts comprising palladium include palladium-alumina, palladium-silica and palladium-carbon; and examples of the catalysts comprising copper include copper, Raney copper, copper-chromium, copper-zinc, copper-chromium-zinc, copper-silica and copper-alumina. The hydrogenolysis reaction may be performed in a liquid phase using a solvent, or in a gas phase. The solvent is required to be substantially inert to the reaction raw materials and the products. The solvent may be a substance present in the residual mixture containing cumyl alcohol to be used. For example, in the case where the residual mixture containing cumyl alcohol contains cumene, the cumene can be used as the solvent without use of other solvent. Although the amount of hydrogen required for the hydrogenolysis reaction may be an equimolar amount of cumyl alcohol, an excessive amount of hydrogen is required because components other than cumyl alcohol to consume hydrogen are usually contained in the raw material. Since the reaction proceeds faster as the partial pressure of hydrogen is increased, the molar ratio of hydrogen/cumyl alcohol is adjusted to usually 1/1 to 20/1, preferably 1/1 to 10/1, more preferably 1/1 to 3/1. The excessive amount of hydrogen remaining after the hydrogenolysis reaction may be recycled for use after separation from the reaction liquid. The hydrogenolysis reaction temperature is usually 0 to 500°C, preferably 50 to 450°C, more preferably 150 to 300°C. The hydrogenolysis reaction pressure is usually 100 to 10000 kPaG, preferably 500 to 4000 kPaG, more preferably 1000 to 2000 kPaG. The hydrogenolysis reaction can be favorably performed in a slurry or a fixed bed mode. In a large-scale industrial operation, it is preferable to use a fixed bed. Further, the hydrogenolysis reaction may be performed by a reaction mode such as a batch process, a semi-continuous process, and a continuous process.

The cumene content of the reaction mixture containing cumene is usually 90 wt% or more per 100 wt% of the reaction mixture containing cumene.

5) Cumene recovery step:

The cumene recovery step of the present invention is a step of obtaining a recovery mixture containing cumene and cymene with a cymene concentration of 100 ppm by weight or more and less than 10000 ppm by weight (the cymene concentration is calculated based on the weight of the recovery mixture containing cumene and cymene obtained in the cumene recovery step) by distilling the reaction mixture containing cumene obtained in the cumene conversion step.

The cymene concentration of the recovery mixture containing cumene and cymene obtained in the cumene recovery step is 100 ppm by weight or more, preferably 110 ppm by weight or more, more preferably 120 ppm by weight or more, still more preferably 130 ppm by weight or more such that the energy required for distillation does not increase excessively in the cumene recovery step. The cymene concentration of the mixture containing cumene and cymene obtained in the cumene recovery step is preferably less than 10000 ppm by weight, preferably 5000 ppm by weight or less, more preferably 3000 ppm by weight or less, still more preferably 1000 ppm by weight or less, in order to more efficiently produce cumene hydroperoxide in the oxidation step and more efficiently produce propylene oxide in the epoxidation step. The cymene concentration is calculated based on the weight of the recovery mixture containing cumene and cymene obtained in the cumene recovery step.

In the cumene recovery step, examples of the method for obtaining the recovery mixture containing cumene and cymene with a cymene concentration of 100 ppm by weight or more and less than 10000 ppm by weight include a method for separating at least a part of cymene in distillation of the reaction mixture containing cumene obtained in the cumene conversion step. Preferably, examples include a method for removing 0.025 wt% or more of cymene from the reaction mixture containing cumene obtained in the cumene conversion step; more preferably, examples include a method for removing 0.04 wt% or more of cymene from the reaction mixture containing cumene obtained in the cumene conversion step; and still more preferably, examples include a method for removing 0.2 wt% or more of cymene from the reaction mixture containing cumene obtained in the cumene conversion step. The weight ratio of the cymene is calculated based on the weight of cymene contained in the reaction mixture containing cumene obtained in the cumene conversion step.

The distillation of the reaction mixture containing cumene obtained in the cumene conversion step is usually operated under conditions in the following ranges: the number of theoretical stages of 10 to 100, a pressure of —100 kPaG to 10000 kPaG, and a temperature of 0 to 500°C. Preferably, the number of theoretical stages is in the range of 10 to 95, the pressure is in the range of -100 kPaG to 5000 kPaG; and a temperature is in the range of 0 to 400°C; and more preferably, the number of theoretical stages is in the range of 10 to 90, the pressure is in the range of —100 kPaG to 3000 kPaG, and a temperature is in the range of 0 to 300°C.

**Examples**

The present invention will be described below in more detail with reference to Examples.

The cymene concentration in each of Examples was measured by the following method using gas chromatography (GC).

[GC measurement conditions]

GC apparatus: Shimadzu GC2014
Column: DB-WAX (30 m, 0.25 mm $\varphi$, 0.25 $\mu$m)
Column temperature: The temperature is maintained at 50°C for 10 minutes, then raised to 220°C at a rate of 4°C/min, and subsequently maintained at 220°C for 48 minutes.
Vaporization chamber temperature/detector temperature: 220°C/230°C
Detector: hydrogen flame ionization detector
Carrier gas: helium
Pressure: 83.7 kPa
Total flow rate: 104.0 mL/min
Column flow rate: 1.00 mL/min
Purge flow rate: 3.0 mL/min
Linear velocity: 25.4 cm/s
Injection method/split ratio: split injection/100:1

Amount injected: 1.0 μL

Cymene concentration analysis

(1) Drawing of calibration curve

[Preparation of solution]

In a 30-mL glass container, 1 g of p-cymene and 19 g of cumene were weighed and well mixed to obtain a sample mother liquid.

In a 20-mL glass container, 2 g of the sample mother liquid was taken and 0.1 g of n-tetradecane was added therein as an internal standard substance to prepare a standard solution 1.

In a 20-mL glass container, 1 g of the standard mother liquid and 4 g of cumene were taken, and 0.1 g of n-tetradecane was added therein to prepare a standard solution 2.

In a 20-mL glass container, 0.2 g of the standard mother liquid and 1.8 g of cumene were taken, and 0.1 g of n-tetradecane was added therein to prepare a standard solution 3.

In a 20-mL glass container, 0.02 g of the standard mother liquid and 1.98 g of cumene were taken, and 0.1 g of n-tetradecane was added therein to prepare a standard solution 4.

[GC measurement]

The standard solutions 1, 2, 3 and 4 were measured under the measurement conditions described in the preceding paragraph so as to draw a calibration curve, with the ordinate representing weight ratio between the sample weight and the internal standard substance, the abscissa representing GC area ratio between the sample and the internal standard substance, so that the slope f of the calibration curve was determined.

(2) Measurement of cymene concentration

[Preparation of sample solution]

In a 20-mL glass container, 2 g of the sample and 0.1 g of n-tetradecane were weighed.

[GC measurement]

Sample solutions were measured under the GC measurement conditions described in the preceding paragraph, and the cymene concentration of the sample was determined according to the following equation.

$W_s$: weight of sample (g)
$W_{is}$: weight of n-tetradecane (internal standard substance) (g)
$A_s$: peak area count of cymene
$A_{is}$: peak area count of n-tetradecane (internal standard substance)
$f$: slope of calibration curve of cymene

$$(\text{Cymene concentration (\%)}) = f \times (A_s/A_{is}) \times (W_{is}/W_s) \times 100$$

[Example 1]

According to the method described in the present specification, an oxidation step, an epoxidation step, a separation step and a cumene conversion step were performed.

In the oxidation step, a reaction mixture containing cumene hydroperoxide was obtained by contacting a mixture containing cumene and cymene with air so as to cause a reaction between cumene in the mixture and oxygen in the air. Subsequently, in the epoxidation step, a reaction mixture containing propylene oxide and cumyl alcohol was obtained by contacting the reaction mixture containing cumene hydroperoxide obtained in the oxidation step with propylene in the presence of a titanium-containing silicon oxide so as to cause a reaction between cumene hydroperoxide in the reaction mixture and propylene. Subsequently, in the separation step, from the reaction mixture containing propylene oxide and cumyl alcohol obtained in the epoxidation step, a mixture containing the propylene oxide was separated to obtain a residual mixture containing the cumyl alcohol. Subsequently, in the cumene conversion step, cumyl alcohol in the residual mixture containing the cumyl alcohol obtained in the separation step was dehydrated in the presence of a catalyst to obtain a mixture containing α-methylstyrene, and subsequently, the mixture containing α-methylstyrene was contacted with hydrogen in the presence of a catalyst to cause a reaction between α-methylstyrene in the mixture and hydrogen, so that a reaction mixture containing cumene was obtained. As a result, in comparison with the mixture containing cumene and cymene used in the oxidation step, the cymene concentration of the reaction mixture containing cumene and cymene after the cumene conversion step was increased by 2 ppm by weight.

Subsequently, the cumene recovery step is performed under the following conditions.

Cumene recovery step: a recovery mixture containing cumene and cymene is obtained by distilling the reaction mixture containing cumene and cymene after the cumene conversion step under a condition for removing 2 wt% of the cymene contained therein (the weight ratio of cymene is calculated based on the weight of cymene contained in the reaction mixture containing cumene in the cumene conversion step).

The recovery mixture containing cumene and cymene obtained in the cumene recovery step is used to occupy 99 wt% of the mixture containing cumene and cymene for use in the oxidation reaction in the oxidation step, and the oxidation step, the epoxidation step, the separation step, the cumene conversion step and cumene recovery step are performed until the cymene concentration became steady. In the steady state, the cymene concentration of the recovery mixture containing cumene and cymene obtained in the cumene recovery step is 100 ppm by weight relative to the recovery mixture containing cumene and cymene.

[Example 2]

According to the method described in the present specification, the oxidation step, the epoxidation step, the separation step and the cumene conversion step were performed.

In the oxidation step, a reaction mixture containing cumene hydroperoxide was obtained by contacting a mixture containing cumene and cymene with air so as to cause a reaction between cumene in the mixture and oxygen in the gas. Subsequently, in the epoxidation step, a reaction mixture containing propylene oxide and cumyl alcohol was obtained by contacting the reaction mixture containing cumene hydroperoxide obtained in the oxidation step with propylene in the presence of a titanium-containing silicon oxide so as to cause a reaction between cumene hydroperoxide in the reaction mixture and propylene. Subsequently, in the separation step, from the reaction mixture containing propylene oxide and cumyl alcohol obtained in the epoxidation step, a mixture containing the propylene oxide was separated to obtain a residual mixture containing the cumyl alcohol. Subsequently, in the cumene conversion step, cumyl alcohol in the residual mixture containing the cumyl alcohol obtained in the separation step was dehydrated in the presence of a catalyst to obtain a mixture containing α-methylstyrene, and subsequently, the mixture containing α-methylstyrene was contacted with hydrogen in the presence of a catalyst to cause a reaction between α-methylstyrene in the mixture and hydrogen, so that a reaction mixture containing cumene was obtained. As a result, in comparison with the mixture containing cumene and cymene used in the oxidation step, the cymene concentration of the reaction mixture containing cumene and cymene after the cumene conversion step was increased by 2 ppm by weight.

Subsequently, the cumene recovery step was performed under the following conditions.

Cumene recovery step: a recovery mixture containing cumene and cymene was obtained by distilling the reaction mixture containing cumene and cymene after the cumene conversion step under a condition for removing 1 wt% of the cymene contained therein (the weight ratio of cymene is calculated based on the weight of cymene contained in the reaction mixture containing cumene in the cumene conversion step).

The recovery mixture containing cumene and cymene obtained in the cumene recovery step was used to occupy 99 wt% of the mixture containing cumene and cymene for use in the oxidation reaction in the oxidation step, and the oxidation step, the epoxidation step, the separation step, the cumene conversion step and cumene recovery step were performed until the cymene concentration became steady. In the steady state, the cymene concentration of the recovery mixture containing cumene and cymene obtained in the cumene recovery step was 200 ppm by weight relative to the recovery mixture containing cumene and cymene.

[Example 3]

The steps are performed in the same manner as in Example 1, except that the reaction mixture containing cumene and cymene after the cumene conversion step is distilled under a condition for removing 0.2 wt% of cymene contained therein. In a steady state, the cymene concentration of the recovery mixture containing cumene and cymene obtained in the recovery step is 1000 ppm by weight relative to the recovery mixture containing cumene and cymene.

[Example 4]

The steps are performed in the same manner as in Example 1, except that the reaction mixture containing cumene and cymene after the cumene conversion step is distilled under a condition for removing 0.04 wt% of cymene contained therein. In a steady state, the cymene concentration of the recovery mixture containing cumene and cymene obtained in the cumene recovery step is 5000 ppm by weight relative to the recovery mixture containing cumene and cymene.

[Comparative Example 1]

The steps are performed in the same manner as in Example 1, except that the reaction mixture containing cumene and cymene after the cumene conversion step is distilled under a condition for removing 0.02 wt% of cymene contained therein. In a steady state, the cymene concentration of the recovery mixture containing cumene and cymene obtained in the cumene recovery step is 10000 ppm by weight relative to the recovery mixture containing cumene and cymene.

[Example 5]

<Oxidation step>

An oxidation reaction was performed under the following conditions.
The recovery mixture containing cumene and cymene obtained in Example 2 and cumene were mixed to prepare a mixture A. The cymene concentration calculated based on the weight of the mixture A was 100 ppm by weight.
Using a continuous flow reaction apparatus having a 1-L glass autoclave as reactor, the following experiment was performed. Into the reactor containing 513 g of the mixture A and 12 g of a sodium carbonate aqueous solution adjusted at a pH of 9, the mixture A was fed at a rate of 126 g/hour, the sodium carbonate aqueous solution adjusted at a pH of 9 was fed at a rate of 5.3 g/hour, and air was fed at a rate of 270 NmL/min, and a continuous flow reaction was performed at a pressure of 0.6 MPaG such that the amount of the liquid in the reactor was maintained at a constant level while withdrawing the liquid and the gas, so that a reaction mixture containing cumene hydroperoxide was obtained. When the reaction achieved a steady state, the reaction temperature was 112.5°C, and the cumene hydroperoxide concentration of the reaction mixture containing cumene hydroperoxide was 16.1 wt% relative to the reaction mixture containing cumene hydroperoxide.

<Epoxidation step>

Subsequently, an epoxidation reaction was performed under the following conditions.
A reaction between 60 g of the reaction mixture containing cumene hydroperoxide obtained in the oxidation step and 33 g of propylene was performed in the presence of 0.5 g of a titanium-containing silicon oxide catalyst manufactured by the method described in Japanese Patent No. 3797107 under conditions at 100°C and 1.8 MPaG for 1.5 hours, so that a reaction mixture containing propylene oxide and cumyl alcohol was obtained. The conversion ratio of cumene hydroperoxide was 94%.

[Example 6]

<Oxidation step>

An oxidation reaction was performed under the following conditions.
The recovery mixture containing cumene and cymene obtained in Example 2 and cymene were mixed to prepare a mixture B, so as to have a cymene concentration of 1000 ppm by weight calculated based on the weight of the mixture to be obtained.
An oxidation reaction was performed in the same manner as in Example 5, except that the mixture A was replaced with the mixture B, and the reaction temperature was adjusted such that a cumene hydroperoxide concentration of the reaction mixture is on the same level with the concentration in Example 5 when the reaction was in a steady state. As a result, the reaction temperature was 114.5°C, and the cumene hydroperoxide concentration of the reaction mixture containing cumene hydroperoxide was 15.3 wt% relative to the reaction mixture containing cumene hydroperoxide.

<Epoxidation step>

Subsequently, an epoxidation reaction was performed under the following conditions.
The epoxidation reaction was performed in the same manner as in Example 5, except that the above reaction mixture containing cumene hydroperoxide obtained in the oxidation step was used. As a result, the conversion ratio of cumene hydroperoxide was 92%.

[Example 7]

<Oxidation step>

The recovery mixture containing cumene and cymene obtained in Example 2 and cymene were mixed to have a cymene concentration of 5000 ppm by weight calculated based on the weight of the mixture to be obtained, so that a mixture C was prepared.

An oxidation reaction was performed in the same manner as in Example 5, except that the mixture A was replaced with the mixture C, and the reaction temperature was adjusted such that a cumene hydroperoxide concentration of the reaction mixture is on the same level with the concentration in Example 5 when the reaction was in a steady state. As a result, the reaction temperature was 116°C, and the cumene hydroperoxide concentration of the reaction mixture containing cumene hydroperoxide was 15.7 wt% relative to the reaction mixture containing cumene hydroperoxide.

<Epoxidation step>

Subsequently, an epoxidation reaction was performed in the same manner as in Example 5, except that the above reaction mixture containing cumene hydroperoxide obtained in the oxidation step was used. As a result, the conversion ratio of cumene hydroperoxide was 91%.

[Comparative Example 2]

<Oxidation step>

The recovery mixture containing cumene and cymene obtained in Example 2 and cymene were mixed to prepare a mixture D, so as to have a cymene concentration of 10000 ppm by weight calculated based on the weight of the mixture to be obtained.

An oxidation reaction was performed in the same manner as in Example 5, except that the mixture A was replaced with the mixture D, and the reaction temperature was adjusted such that a cumene hydroperoxide concentration of the reaction mixture is a same level with the concentration in Example 5 when the reaction was in a steady state. As a result, the reaction temperature was 116.3°C, and the cumene hydroperoxide concentration of the reaction mixture containing cumene hydroperoxide was 15.5 wt% relative to the reaction mixture containing cumene hydroperoxide.

<Epoxidation step>

Subsequently, the epoxidation reaction was performed in the same manner as in Example 5, except that the above reaction mixture containing cumene hydroperoxide obtained in the oxidation step was used. As a result, the conversion ratio of cumene hydroperoxide was 87%.

Example 5, Example 6, Example 7, and Comparative Example 2 shows the oxidation reaction temperature in the oxidation step when the cymene concentration of the mixture containing cumene and cymene is different from each other and the cumene hydroperoxide concentration of the reaction mixture is adjusted to be a same level to each other in a state in which the reaction has become steady. From the results in Example 5, Example 6, Example 7, and Comparative Example 2, it is found that the reaction temperature can be lowered to obtain cumene hydroperoxide with an equivalent concentration for an equivalent residence time in Examples, with a faster rate of the oxidation reaction.

As described above, the present invention provides a method for producing propylene oxide by recycling of cumene for use as a raw material, the method having excellent features that allows propylene oxide to be more efficiently produced with an improved oxidation reaction rate in an oxidation step and an improved conversion ratio of cumene hydroperoxide in an epoxidation step.

**Industrial Applicability**

The present invention can be used in industrial production of propylene oxide.

**Claims**

**1.** A method for producing propylene oxide, the method comprising the following steps 1) to 5):

1) oxidation step: a step of obtaining a reaction mixture containing cumene hydroperoxide by contacting a mixture containing cumene and cymene with an oxygen-containing gas to cause a reaction between cumene in the mixture and oxygen in the gas;

2) epoxidation step: a step of obtaining a reaction mixture containing propylene oxide and cumyl alcohol by contacting the reaction mixture containing cumene hydroperoxide obtained in the oxidation step with propylene in the presence of a catalyst to cause a reaction between cumene hydroperoxide in the reaction mixture and propylene;

3) separation step: a step of obtaining a residual mixture containing cumyl alcohol by separating a mixture containing propylene oxide from the reaction mixture containing propylene oxide and cumyl alcohol obtained in the epoxidation step;

4) cumene conversion step: a step of obtaining a reaction mixture containing cumene by converting cumyl alcohol in the residual mixture containing cumyl alcohol obtained in the separation step into cumene; and

5) cumene recovery step: a step of obtaining a recovery mixture containing cumene and cymene with a cymene concentration, in a steady state, of 100 ppm by weight or more and 5000 ppm by weight or less (the cymene concentration is calculated based on the weight of the recovery mixture containing cumene and cymene obtained in the cumene recovery step) by distilling the reaction mixture containing cumene obtained in the cumene conversion step, wherein the distillation is performed under a condition for removing 0.025 wt% or more of cymene contained in the reaction mixture containing cumene obtained in the cumene conversion step (the weight ratio of cymene is calculated based on the weight of cymene contained in the reaction mixture containing cumene obtained in the cumene conversion step); wherein, at least a part of the mixture containing cumene and cymene in the oxidation step is the recovery mixture containing cumene and cymene obtained in the cumene recovery step.

2. The method for producing propylene oxide according to claim 1, wherein the cumene conversion step is a step of obtaining a reaction mixture containing cumene by dehydrating cumyl alcohol in the residual mixture containing cumyl alcohol obtained in the separation step in the presence of a catalyst to obtain a mixture containing $\alpha$-methylstyrene, and then contacting the mixture containing $\alpha$-methylstyrene with hydrogen in the presence of a catalyst to cause a reaction between $\alpha$-methylstyrene in the mixture and hydrogen.

3. The method for producing propylene oxide according to claim 1, wherein the cumene conversion step is a step of obtaining a reaction mixture containing cumene by contacting the residual mixture containing cumyl alcohol obtained in the separation step with hydrogen in the presence of a catalyst to cause a reaction between cumyl alcohol in the residual mixture and hydrogen.

4. The method for producing propylene oxide according to any one of claims 1 to 3, wherein the cymene concentration of the reaction mixture containing cumene hydroperoxide in the epoxidation step is 100 ppm by weight or more and less than 10000 ppm by weight (the cymene concentration is calculated based on the weight of the reaction mixture containing cumene hydroperoxide).

5. The method for producing propylene oxide according to any one of claims 1 to 4, wherein the catalyst in the epoxidation step is a solid catalyst.

6. The method for producing propylene oxide according to any one of claims 1 to 5, wherein the catalyst in the epoxidation step comprises a titanium-containing silicon oxide.

7. The method for producing propylene oxide according to any one of claims 1 to 6, further comprising the following step: propylene oxide purification step: a step of obtaining purified propylene oxide by distilling the mixture containing propylene oxide separated in the separation step.

**Patentansprüche**

1. Verfahren zur Herstellung von Propylenoxid, wobei das Verfahren die folgenden Schritte 1) bis 5) umfasst:

1) Oxidationsschritt: ein Schritt, bei dem eine Reaktionsmischung erhält, die Cumolhydroperoxid enthält, indem man eine Mischung, die Cumol und Cymol enthält, mit einem sauerstoffhaltigen Gas in Kontakt bringt, um eine Reaktion zwischen Cumol in der Mischung und Sauerstoff in dem Gas zu bewirken;

2) Epoxidationsschritt: ein Schritt, bei dem man eine Reaktionsmischung erhält, die Propylenoxid und Cumylalkohol enthält, indem man die im Oxidationsschritt erhaltene Reaktionsmischung, die Cumolhydroperoxid enthält, mit Propylen in Gegenwart eines Katalysators in Kontakt bringt, um eine Reaktion zwischen Cumolhydroperoxid in der Reaktionsmischung und Propylen zu bewirken;

3) Abtrennungsschritt: ein Schritt bei dem man eine Restmischung erhält, das Cumylalkohol enthält, indem eine Propylenoxid enthaltende Mischung, von der im Epoxidationsschritt erhaltenen Reaktionsmischung, die Propylenoxid und Cumylalkohol enthält, abtrennt;

4) Cumolumwandlungsschritt: ein Schritt, bei dem man eine Reaktionsmischung erhält, die Cumol enthält, indem man Cumylalkohol in der im Abtrennungsschritt erhaltenen Restmischung, die Cumylalkohol enthält, in Cumol umwandelt; und

5) Cumolrückgewinnungsschritt: ein Schritt, bei dem man eine Rückgewinnungsmischung erhält, die Cumol und Cymol enthält, mit einer Cymolkonzentration in einem stationären Zustand von 100 ppm, bezogen auf das Gewicht, oder mehr und 5000 ppm, bezogen auf das Gewicht, oder weniger (wobei die Cymolkonzentration berechnet wird auf Basis des Gewichts der Cumol und Cymol enthaltenden Rückgewinnungsmischung, die im Cumolrückgewinnungsschritt erhalten wird), indem man die im Cumolumwandlungsschritt erhaltene Reaktionsmischung, die Cumol enthält, destilliert, wobei die Destillation unter einer Bedingung zum Entfernen von 0,025 Gew.-% oder mehr des in der im Cumolumwandlungsschritt erhaltenen Reaktionsmischung, die Cumol enthält, enthaltenen Cymols (wobei das Gewichtsverhältnis an Cymol auf Basis des Gewichts an Cymol berechnet wird, das in der im Cumolumwandlungsschritt erhaltenen Reaktionsmischung, die Cumol enthält, enthalten ist) durchgeführt wird; wobei

mindestens ein Teil der Cumol und Cymol enthaltenden Mischung im Oxidationsschritt, die im Cumolrückgewinnungsschritt erhaltene Rückgewinnungsmischung ist, die Cumol und Cymol enthält.

2. Verfahren zur Herstellung von Propylenoxid nach Anspruch 1, wobei der Cumolumwandlungsschritt ein Schritt ist, bei dem eine Cumol enthaltende Reaktionsmischung erhalten wird, indem Cumylalkohol in der Cumylalkohol enthaltenden Restmischung, die im Abtrennungsschritt erhalten wird, in Gegenwart eines Katalysators dehydratisiert wird, um ein Mischung zu erhalten, die α-Methylstyrol enthält, und dann die α-Methylstyrol enthaltende Mischung in Gegenwart eines Katalysators mit Wasserstoff in Kontakt gebracht wird, um eine Reaktion zwischen α-Methylstyrol in der Mischung und Wasserstoff zu bewirken.

3. Verfahren zur Herstellung von Propylenoxid nach Anspruch 1, wobei der Cumolumwandlungsschritt ein Schritt ist, bei dem eine Cumol enthaltende Reaktionsmischung erhalten wird, indem die im Abtrennungsschritt erhaltene Restmischung, die Cumylalkohol enthält, in Gegenwart eines Katalysators mit Wasserstoff in Kontakt gebracht wird, um eine Reaktion zwischen Cumylalkohol in der Restmischung und Wasserstoff zu bewirken.

4. Verfahren zur Herstellung von Propylenoxid nach einem der Ansprüche 1 bis 3, wobei die Cymolkonzentration der Cumolhydroperoxid enthaltenden Reaktionsmischung im Epoxidationsschritt 100 ppm, bezogen auf das Gewicht, oder mehr und weniger als 10000 ppm, bezogen auf das Gewicht, beträgt (wobei die Cymolkonzentration basierend auf dem Gewicht der Cumolhydroperoxid enthaltenden Reaktionsmischung berechnet wird).

5. Verfahren zur Herstellung von Propylenoxid nach einem der Ansprüche 1 bis 4, wobei der Katalysator im Epoxidationsschritt ein fester Katalysator ist.

6. Verfahren zur Herstellung von Propylenoxid nach einem der Ansprüche 1 bis 5, wobei der Katalysator im Epoxidationsschritt ein titanhaltiges Siliciumoxid umfasst.

7. Verfahren zur Herstellung von Propylenoxid nach einem der Ansprüche 1 bis 6, ferner umfassend den folgenden Schritt:
Propylenoxidreinigungsschritt: ein Schritt, bei dem gereinigtes Propylenoxid erhalten wird, indem die im Abtrennungsschritt abgetrennte Mischung, die Propylenoxid enthält, destilliert wird.

**Revendications**

1. Procédé pour la production d'oxyde de propylène, le procédé comprenant les étapes 1) à 5) suivantes :

1) étape d'oxydation : une étape d'obtention d'un mélange réactionnel contenant de l'hydroperoxyde de cumène par mise en contact d'un mélange contenant du cumène et cymène avec un gaz contenant de l'oxygène pour occasionner une réaction entre le cumène dans le mélange et l'oxygène dans le gaz ;

2) étape d'époxydation : une étape d'obtention d'un mélange réactionnel contenant de l'oxyde de propylène et alcool cumylique par mise en contact du mélange réactionnel contenant de l'hydroperoxyde de cumène obtenu dans l'étape d'oxydation avec du propylène en présence d'un catalyseur pour occasionner une réaction entre l'hydroperoxyde de cumène dans le mélange réactionnel et le propylène ;

3) étape de séparation : une étape d'obtention d'un mélange résiduel contenant de l'alcool cumylique par séparation d'un mélange contenant de l'oxyde de propylène du mélange réactionnel contenant de l'oxyde de propylène et alcool cumylique obtenu dans l'étape d'époxydation ;

4) étape de conversion de cumène : une étape d'obtention d'un mélange réactionnel contenant du cumène par conversion d'alcool cumylique dans le mélange résiduel contenant de l'alcool cumylique obtenu dans l'étape de séparation en cumène ; et

5) étape de récupération de cumène : une étape d'obtention d'un mélange de récupération contenant du cumène et cymène avec une concentration en cymène, dans un état permanent, de 100 ppm en masse ou plus et 5 000 ppm en masse ou moins (la concentration en cymène est calculée sur la base de la masse du mélange de récupération contenant du cumène et cymène obtenu dans l'étape de récupération de cumène) par distillation du mélange réactionnel contenant du cumène obtenu dans l'étape de conversion de cumène, dans lequel la distillation est réalisée dans une condition d'élimination de 0,025 % en masse ou plus de cymène contenu dans le mélange réactionnel contenant du cumène obtenu dans l'étape de conversion de cumène (le rapport massique de cymène est calculé sur la base de la masse de cymène contenu dans le mélange réactionnel contenant du cumène obtenu dans l'étape de conversion de cumène) ; dans lequel

au moins une partie du mélange contenant du cumène et cymène dans l'étape d'oxydation est le mélange de récupération contenant du cumène et cymène obtenu dans l'étape de récupération de cumène.

2. Procédé pour la production d'oxyde de propylène selon la revendication 1, dans lequel l'étape de conversion de cumène est une étape d'obtention d'un mélange réactionnel contenant du cumène par déshydratation d'alcool cumylique dans le mélange résiduel contenant de l'alcool cumylique obtenu dans l'étape de séparation en présence d'un catalyseur pour obtenir un mélange contenant de l'a-méthylstyrène, et puis mise en contact du mélange contenant l'a-méthylstyrène avec de l'hydrogène en présence d'un catalyseur pour occasionner une réaction entre l'a-méthylstyrène dans le mélange et l'hydrogène.

3. Procédé pour la production d'oxyde de propylène selon la revendication 1, dans lequel l'étape de conversion de cumène est une étape d'obtention d'un mélange réactionnel contenant du cumène par mise en contact du mélange résiduel contenant de l'alcool cumylique obtenu dans l'étape de séparation avec de l'hydrogène en présence d'un catalyseur pour occasionner une réaction entre l'alcool cumylique dans le mélange résiduel et l'hydrogène.

4. Procédé pour la production d'oxyde de propylène selon l'une quelconque des revendications 1 à 3, dans lequel la concentration en cymène du mélange réactionnel contenant de l'hydroperoxyde de cumène dans l'étape d'époxydation est de 100 ppm en masse ou supérieure et inférieure à 10 000 ppm en masse (la concentration en cymène est calculée sur la base de la masse du mélange réactionnel contenant l'hydroperoxyde de cumène).

5. Procédé pour la production d'oxyde de propylène selon l'une quelconque des revendications 1 à 4, dans lequel le catalyseur dans l'étape d'époxydation est un catalyseur solide.

6. Procédé pour la production d'oxyde de propylène selon l'une quelconque des revendications 1 à 5, dans lequel le catalyseur dans l'étape d'époxydation comprend un oxyde de silicium contenant du titane.

7. Procédé pour la production d'oxyde de propylène selon l'une quelconque des revendications 1 à 6, comprenant de plus l'étape suivante :

étape de purification d'oxyde de propylène : une étape d'obtention d'oxyde de propylène purifié par distillation du mélange contenant de l'oxyde de propylène séparé dans l'étape de séparation.

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005097212 A
- JP 3731384 B
- JP 3797107 B
- US 2005014960 A
- US 2007260074 A
- US 5783167 A
- JP H7300312 A
- CN 101348472 B
- CN 101307039 B
- CN 101279960 B
- CN 102311363 B
- CN 102872847 B
- CN 103030611 B
- JP 2003032745 A

**Non-patent literature cited in the description**

- *Nature,* 1994, vol. 368, 321
- *Chemistry of Material,* 2002, vol. 14, 1657
- *Chemistry Letters,* 2000, 774